# EUROPEAN PATENT APPLICATION

(11) **EP 4 238 561 A1**
(43) Date of publication of application: **06.09.2023**
(21) Application number: 21897043.2
(22) Date of filing: 24.11.2021
(51) Int. Cl.: A61K 31/351, A61P 15/00

(54) **USE OF ZANAMIVIR IN PREPARATION OF DRUG FOR TREATING OR PREVENTING PREECLAMPSIA**

(30) Priority: 24.11.2020 CN 202011331782; 01.12.2020 CN 202011388482
(71) Applicant: Shenzhen Evergreen Therapeutics Co., Ltd., Shenzhen, Guangdong 518038 (CN)
(72) Inventor: DU, Tao Tom, Shenzhen, Guangdong 518038 (CN); HU, Tao, Shenzhen, Guangdong 518038 (CN); DU, Xin, Shenzhen, Guangdong 518038 (CN)
(74) Representative: J A Kemp LLP
(86) International application number: PCT/CN2021/132895
(87) International publication number: WO 2022/111543

(57) **Abstract**

Provided are a use of zanamivir in preparation of a drug for treating or preventing preeclampsia and a corresponding drug and treatment method. Zanamivir can effectively improve the symptoms of hypertension, proteinuria, increased creatinine, neuraminidase and sialic acid of preeclampsia, increase the NO level, and improve pregnancy outcome. A combination of zanamivir and hydroxyprogesterone caproate shows a better effect.

## Description

### CROSS REFERENCE TO RELATED APPLICATIONS

This application claims priority to Chinese patent application No. 202011331782.9, filed with the Chinese Patent Office on November 24, 2020, entitled "Method of treating pre-eclampsia," the entire contents of which are incorporated by reference in this application.

This application claims priority to a Chinese patent application filed with the Chinese Patent Office on December 01, 2020, Application No. 202011388482.4, entitled "Method of Treating Pre-eclampsia", the entire contents of which are incorporated by reference in this application.

This application claims priority to the Chinese patent application filed with the Chinese Patent Office on November 24, 2021, Application No. 202111400709.7, entitled "Use of Zanamivir in the Preparation of Drugs for the Treatment or Prevention of Pre-eclampsia" , the entire contents of which are incorporated by reference in this application.

### TECHNICAL FIELD

This application provides the use of zanamivir in the preparation of drugs for the treatment or prevention of pre-eclampsia and the corresponding drugs and methods of treatment.

### BACKGROUND

Pre-eclampsia, also known as pre-eclampsia or toxemia of pregnancy, is a clinical syndrome with hypertension, proteinuria and edema as the main symptoms, accompanied by headache, ophthalmia, malignancy, vomiting, abdominal discomfort, etc., that appears after 20 weeks of gestation . Pre-eclampsia not only affects the development of the fetus, but also often endangers the life of the mother. According to statistics, pre-eclampsia affects approximately 8.5 million pregnant women worldwide each year and causes approximately 63,000 maternal and 500,000 perinatal deaths, and is an important factor in maternal and perinatal morbidity and mortality. There is also evidence that the prevalence of pre-eclampsia is higher in developing countries, with an estimated 9.4% in China. The pathophysiological process of the disease is broadly divided into 2 stages: 1) abnormal placental formation, especially impaired diastole of the small uterine spiral arteries, and affects the maternal blood supply to the fetus. The reduced placental perfusion then activates the release of associated cytokines from the placenta and causes systemic hemodynamic changes. 2) Maternal clinical symptoms, mainly circulatory disturbances caused by maternal vascular endothelial cell dysfunction, including altered vascular reactivity, activation of the coagulation cascade and disruption of vascular integrity. Preeclampsia affects most of the maternal organs, but mainly the kidneys, liver and brain.

Although there are numerous preclinical and clinical studies on preeclampsia, its pathogenesis is known to be complex but not yet clear, and the corresponding treatment is quite scarce, generally only symptomatic hypotensive, diuretic, sedative treatment, etc. Magnesium sulfate can be used to relieve spasticity symptoms when severe eclampsia appears, and termination of pregnancy is still the most effective clinical treatment. The etiology of pre-eclampsia may be based on polygenic genetic factors, susceptibility factors leading to abnormal cellular immunity, placental and trophoblast ischemia and hypoxia, and abnormal oxidative stress response, causing secondary systemic vascular endothelial cell damage and leading to the development of the disease. In view of this, a series of mechanistic theories including placental or trophoblast ischemia, oxidative stress, abnormal immune regulation, genetic theory and vascular endothelial damage have been proposed to elucidate the pathogenesis of pre-eclampsia. The pathophysiological basis for the pathogenesis of preeclampsia is usually considered to be the trophoblastic ischemia and hypoxia and vascular endothelial damage due to impaired recasting of small placental spiral arteries and shallow placental implantation. Numerous studies have shown that the release of placental and vascular-derived circulating factors due to ischemia and hypoxia, including vascular endothelial growth factor (VEGF), soluble vascular endothelial growth factor-1 (sFlt-1), autoantibodies to angiotensin II receptor 1 and tumor necrosis factor-α (TNF-α), interleukin 1 (IL-1) and IL-6, and pentraxin-3 ( PTX3) can cause maternal vascular endothelial function impairment through different pathways. In addition, the ratio of vasodilators to vasoconstrictors in the blood is imbalanced in patients with pre-eclampsia, and damage to the vascular endothelium increases the sensitivity of blood vessels to vasoconstrictors and decreases the sensitivity to vasodilators, leading to systemic small artery spasms. Patients with pre-existing hypertension, diabetes mellitus, obesity and hyperlipidemia may have oxidative stress and abnormal endothelial function before pregnancy and are more likely to develop pre-eclampsia after pregnancy.

Vascular endothelial cells lie flat on the luminal surface of blood vessels and are direct receptors and responders of flow shear. Endothelial cells convert extracellular mechanical signals into intracellular signals through various pathways, which in turn trigger downstream biochemical responses. The vascular endothelial cell glycocalyx is a villi-like polysaccharide protein complex structure located in the parietal membrane of endothelial cells between the vessel wall and the blood, acting as a dynamic natural barrier on the endothelial cell surface and mediating endothelial cell force transduction functions. The glycocalyx of vascular endothelial cells is a highly negatively charged endothelial cell surface layer composed of oligosaccharide chain glycoproteins with terminal sialic acid residues and proteoglycans containing glycosaminoglycan side chains. In addition to acting as a selective permeability barrier in the vascular wall, regulating the rheological properties of the microcirculation, and participating in vascular protection and signaling, there is substantial evidence that the endothelial cell glycocalyx is also involved in the perception and transmission of flow shear. Using a selective enzymatic degradation approach, Manolis et al. treated bovine thoracic aortic endothelial cells by selectively degrading sialic acid, heparan sulfate, chondroitin sulfate, and hyaluronic acid in the glycocalyx simultaneously and found that flow shear-induced nitric oxide (NO) synthesis was blocked, suggesting that the intact glycocalyx plays an important role in shear-induced NO synthesis. The study also confirmed that when the glycocalyx was intact, mobile shear could be transmitted to the actin cytoskeleton via core proteins or directly to the cell membrane via core proteins, thereby mediating downstream signaling, such as NO production and cytoskeletal reorganization. In contrast, when the glycocalyx is impaired, the normal function of the vascular endothelium is compromised. A recent clinical study found increased glycocalyx degradation in patients with early-onset pre-eclampsia, accompanied by increased levels of heparan sulfate proteoglycans and hyaluronic acid, suggesting that endothelial glycocalyx degradation is strongly associated with the development of early-onset pre-eclampsia.

The degradation of the vascular endothelial glycocalyx is usually mediated by a variety of specific enzymes. Neuraminidases, also known as sialidases, can act on oligosaccharide chain glycoproteins containing sialic acid residues at the end of the glycocalyx to hydrolyze their Ketocoside bonds and release sialic acid, ultimately causing glycocalyx degradation and corresponding impairment of vascular endothelial function. It has been documented that elevated levels of neuraminidase can be detected in the blood of patients with some cardiovascular system-related diseases such as type II diabetes, and that inhibition of neuraminidase can improve vascular endothelial function as well as the function of the entire cardiovascular system by inhibiting glycocalyx degradation. Given that there is no effective drug for the prevention and treatment of pre-eclampsia, there is a great need for drugs for the prevention and treatment of this disease in the field, and drugs that inhibit neuraminidase provide a new idea and means for the development of drugs for the prevention and treatment of pre-eclampsia.

### SUMMARY OF THE INVENTION

On the one hand, this application provides the use of zanamivir in the preparation of drugs for the treatment or prevention of pre-eclampsia .

Said zanamivir includes the compound zanamivir and medically or pharmaceutically acceptable derivatives or salts thereof.

Further, said pharmaceutical dosage form is an injectable, an oral formulation or a topical formulation; preferably an injectable, an oral formulation; particularly preferably an injectable.

Further, said injectables include injections, powder injections; oral preparations include tablets, capsules, oral liquids, throat or nasal sprays, and topical preparations include ointments, sprays, patches.

Further, described as treating or preventing pre-eclampsia are improving headache, ophthalmia, nausea, vomiting, abdominal discomfort, hypertension, proteinuria, edema, liver and kidney function, thrombocytopenia and/or coagulation system abnormalities; elevated creatinine, neuraminidase and sialic acid, and lower blood NO levels; and improving pregnancy outcomes.

On the other hand, the present application provides methods for treating or preventing pre-eclampsia, including administering zanamivir to patients at risk of or suffering from pre-eclampsia.

Further, said zanamivir dosage form is an injectable, oral formulation or topical formulation; preferably an injectable, oral formulation; particularly preferably an injectable.

Said injection includes injection, powder injection; oral preparations include tablets, capsules, oral liquid, throat or nasal spray, topical preparations include ointments, sprays, patches.

Further, said treatment or prevention of pre-eclampsia is improvement of headache, ophthalmia, nausea, vomiting, abdominal discomfort, hypertension, proteinuria, edema, liver and kidney function, thrombocytopenia and/or coagulation system abnormalities; elevated creatinine, neuraminidase and sialic acid, and reduced blood NO levels; and improvement of pregnancy outcome.

Further, said method of administration of zanamivir may be selected from intramuscular injection, intravenous injection, intravenous infusion, preferably intramuscular injection; administered 1-7 times per week, preferably 1-4 times per week; more preferably 1-2 times per week; duration of administration 1-30 days, preferably 1-7 days, more preferably 1-7 days.

On the other hand, the present application provides drugs for the treatment or prevention of pre-eclampsia.

Further, said pharmaceutical dosage form is an injection, an oral formulation or a topical formulation; preferably an injection, an oral formulation; particularly preferably an injection; further preferably an intramuscular injection.

Further, said drug contains instructions, which document the administration of zanamivir as being administered 1-7 times per week, preferably 1-4 times; more preferably 1-2 times; for a duration of administration of 1-30 days, preferably 1-7 days, more preferably 1-7 days.

Further, described as treating or preventing pre-eclampsia are improving headache, ophthalmia, nausea, vomiting, abdominal discomfort, hypertension, proteinuria, edema, liver and kidney function, thrombocytopenia and/or coagulation system abnormalities; elevated creatinine, neuraminidase and sialic acid, and reduced blood NO levels; and improving pregnancy outcomes.

Further, the method or drug of the present application is administered at a dose of 20-40 mg/day,90 mg/kg or 120 mg/kg, preferably 20 mg/day.

Further, said drug further comprises pharmaceutically acceptable excipients, said pharmaceutically acceptable excipients including but not limited to solvents, co-solvents, stabilizers, dispersants, viscosity modifiers, antioxidants, sweeteners, binders, gas generators.

Further, zanamivir is the sole active ingredient in the method or drug; or other active ingredients are also included in the method for administration or other active ingredients are also included in the drug; said other active ingredients include, but are not limited to, hypotensive agents, protein supplements, anti-vertigo agents, cardiovascular disease drugs.

On the other hand, the present application provides the use of neuraminidase inhibitors in the preparation of drugs for the treatment or prevention of pre-eclampsia.

Said neuraminidase inhibitor comprises the compound neuraminidase inhibitor and a medically or pharmaceutically acceptable derivative or salt thereof.

Further, said pharmaceutical dosage form is an injectable, an oral formulation or a topical formulation; preferably an injectable, an oral formulation; particularly preferably an injectable.

Further, said injection includes injection, powder injection; oral preparations include tablets, capsules, oral liquid, throat or nasal spray, and topical preparations include ointment, spray, patch.

Further, said treatment or prevention of pre-eclampsia is improvement of headache, ophthalmia, nausea, vomiting, abdominal discomfort, hypertension, proteinuria, edema, liver and kidney function, thrombocytopenia and/or coagulation system abnormalities; elevated creatinine, neuraminidase and sialic acid, and reduced blood NO levels; and improvement of pregnancy outcome.

On the other hand, the present application provides methods for treating or preventing pre-eclampsia, including administering neuraminidase inhibitors to patients at risk of or suffering from pre-eclampsia.

Further, said neuraminidase inhibitor dosage form is an injection, an oral formulation or a topical formulation; preferably an injection, an oral formulation; particularly preferably an injection.

Said injection includes injection, powder injection; oral preparations include tablets, capsules, oral liquid, throat or nasal spray, topical preparations include ointments, sprays, patches.

Further, said treatment or prevention of pre-eclampsia is improvement of headache, ophthalmia, nausea, vomiting, abdominal discomfort, hypertension, proteinuria, edema, liver and kidney function, thrombocytopenia and/or coagulation system abnormalities; elevated creatinine, neuraminidase and sialic acid, and reduced blood NO levels; and improvement of pregnancy outcome.

Further, said neuraminidase inhibitor may be administered by intramuscular injection, intravenous injection, intravenous infusion, preferably intramuscular; administered 1-7 times per week, preferably 1-4 times; more preferably 1-2 times; and administered for a duration of 1-30 days, preferably 1-7 days, more preferably 1-7 days.

On the other hand, the present application provides drugs for the treatment or prevention of pre-eclampsia.

Further, said pharmaceutical dosage form is an injection, an oral formulation or a topical formulation; preferably an injection, an oral formulation; particularly preferably an injection; further preferably an intramuscular injection.

Further, said medication comprises instructions, which document the administration of the neuraminidase inhibitor as being administered 1-7 times per week, preferably 1-4 times; more preferably 1-2 times; and the duration of administration 1-30 days, preferably 1-7 days, more preferably 1-7 days.

Further, said treatment or prevention of pre-eclampsia is improvement of headache, ophthalmia, nausea, vomiting, abdominal discomfort, hypertension, proteinuria, edema, liver and kidney function, thrombocytopenia and/or coagulation system abnormalities; elevated creatinine, neuraminidase and sialic acid, and reduced blood NO levels; and improvement of pregnancy outcome.

Further, the method or drug of the present application is administered at a dose of 20-40 mg/day,90 mg/kg or 120 mg/kg, preferably 20 mg/day.

Further, said drug further comprises pharmaceutically acceptable excipients, said pharmaceutically acceptable excipients including but not limited to solvents, co-solvents, stabilizers, dispersants, viscosity modifiers, antioxidants, sweeteners, binders, gas generators.

Further, the neuraminidase inhibitor is the sole active ingredient in the method or drug; or other active ingredients are also included in the method for administration or other active ingredients are also included in the drug; said other active ingredients include, but are not limited to, hypotensive agents, protein supplements, anti-vertigo agents, cardiovascular disease drugs.

Thezanamivir described in this application is not limited to the chemical structure of CAS No. 139110-90-8 (i.e., 5-acetamido-4-[(aminoiminomethyl)-amino]-2,6-hydro-3,4,5-trideoxy-D-propanetriolyl-D-galactos-2-enolonic acid, or 4-guanidino-2-deoxy-2,3-didehydro-N-acetylneuraminic acid), and also contains its medically or pharmaceutically acceptable derivatives or salts . A neuraminidase inhibitor in this application is a compound or composition that acts as an inhibitor of neuraminidase and includes various neuraminidase inhibitors known and under investigation in the art, including but not limited to zanamivir.

In this application hydroxyprogesterone caproate is also known as 17-alpha hydroxyprogesterone caproate, 17-HPC, 17-hydroxyprogesterone caproate, 17-hydroxyhexogestrel, CAS No. 630-56-8.

Pre-eclampsia as described in this application refers to the clinical syndrome of hypertension, proteinuria and edema as the main symptoms during pregnancy, accompanied by headache, ophthalmia, nausea, vomiting, abdominal discomfort , etc. The main symptoms also include elevated creatinine, neuraminidase and sialic acid and reduced blood NO levels , the specific symptoms of which include but are not limited to the above.

Zanamivir in this application can be administered as the sole active ingredient for the treatment of pre-eclampsia or to prepare the corresponding drug, or it can be administered in combination or prepared as a pharmaceutical combination or pharmaceutical composition with a known or investigational drug for the treatment of pre-eclampsia or pre-eclampsia-related symptoms. These drugs include, but are not limited to, blood pressure lowering drugs, protein supplements, anti-vertigo drugs, cardiovascular disease drugs , etc.

The mode of administration of zanamivir injection in this application can be intramuscular injection, intravenous injection, intravenous infusion, etc., preferably intramuscular injection .

Depending on the dosage form and the condition, the frequency of zanamivir administration in this application can be 1-7 times per week, preferably 1-4 times, more preferably 1-2 times; the duration of administration can be 1-30 days, preferably 1-14 days, more preferably 1-7 days.

The dosage of zanamivir in this application is not limited to the scope of the specification and claims, and the clinician may adjust the dosage according to the patient's condition and the drug dosage form.

Available dosage forms of zanamivir in this application include, but are not limited to, injectable formulations such as injections, powder injections, etc., oral formulations such as tablets, capsules, oral liquids, etc., throat or nasal sprays, topical formulations such as ointments, sprays, patches, etc.; preferably injectable formulations , oral formulations; and particularly preferably injectable formulations .

The and improved pregnancy outcomes described in this application include, but are not limited to, miscarriage and fetal/infant health problems associated with pre-eclampsia or blood pressure.

The person skilled in the art can select suitable excipients for the drugs covered by this application based on general knowledge in the field of pharmacy. The types of excipients available include, but are not limited to, solvents, co-solvents, stabilizers, dispersants, viscosity modifiers, antioxidants, sweeteners, binders, gas generators , etc.

In the present invention, neuraminidase inhibitors such as zanamivir may be administered at a dosage of 90 mg/kg-120 mg/kg; preferably 120 mg/kg.

The results showed that zanamivir improved hypertension as well as proteinuria symptoms in pre-eclampsia and reduced creatinine levels in urine; zanamivir reduced neuraminidase and sialic acid levels in blood and increased NO levels in blood. Zanamivir in combination with hydroxyprogesterone caproate has shown superior preventive and therapeutic effects in preeclampsia. It provides a new idea and means of drug development for the prevention and treatment of pre-eclampsia.

### BRIEF DESCRIPTION OF THE DRAWINGS

Figure 1 shows the mean arterial pressure on day 12 of gestation for each group of pregnant rats ; it shows that zanamivir (120 mg/kg) significantly reduced the L-NAME-induced increase in mean arterial pressure in pregnant rats (data are mean ± standard error, N = 9-22, * p<0.05).
Figure 2 shows a graph of diastolic blood pressure on day 12 of gestation for each group of pregnant rats at ; it shows that zanamivir (120 mg/kg) significantly reduced the L-NAME-induced increase in diastolic blood pressure in pregnant rats (data are mean ± standard error, N = 9-22, * p<0.05).
Figure 3 shows the systolic blood pressure on day 12 of gestation in the groups of pregnant rats at ; it shows that zanamivir (120 mg/kg) significantly reduced the L-NAME-induced increase in systolic blood pressure in pregnant rats (data are mean ± standard error, N = 9-22, ** p<0.01).
Figure 4 shows a graph of 24-hour urinary protein in each group of pregnant rats on days 17-18 of gestation; it shows that zanamivir significantly reduces the L-NAME-induced elevation of 24-hour urinary protein in pregnant rats (data are mean ± standard error, N = 5-22, ** p<0.01, *** p<0.001).
Figure 5 shows the litter size in each group; zanamivir significantly improved the reduction of litter size in pregnant rats caused by L-NAME (data are mean ± standard error, N = 9-16, * p<0.05).

### DETAILED DESCRIPTION

### Example 1 Establishment of animal model of pre-eclampsia

Pregnant CD-1 mice (age 8-10 weeks) were randomly divided into control and pre-eclampsia model groups. On days 7-16 of the gestational cycle, the model group mice were induced with preeclampsia by subcutaneous injection of 50 mg/kg of N-nitro-L-arginine methyl ester (L-NAME) daily. For the control group, an equal volume of the solvent was injected subcutaneously.

### Example 2 Experimental grouping

| Group | Moulding (Day 7-16 of pregnancy, administered daily) | Treatment (Days 1-18 of pregnancy, administered daily) |
|---|---|---|
| Non-pregnant control group (N=22) | normal saline | normal saline |
| Pregnant control group (N=16) | normal saline | normal saline |
| Pregnancy model group (N=18) | L-NAME (50 mg/kg, subcutaneous injection) | normal saline |
| Pregnancy treatment group (N=9) | L-NAME (50 mg/kg, subcutaneous injection) | Zanamivir (90 mg/kg, tail vein injection) |
| Pregnancy treatment group (N=12) | L-NAME (50 mg/kg, subcutaneous injection) | Zanamivir (120 mg/kg, tail vein injection) |

### Example 3 Evaluation of treatment effect

In animal models of L-NAME-induced pre-eclampsia, symptoms of hypertension appear two days after the first administration of L-NAME and persist until at least five days after the first administration of L-NAME, according to the literature. The appearance of proteinuric symptoms was relatively late. Therefore, in this experiment, we focused on monitoring the blood pressure of pregnant rats on day 12 of gestation (i.e., five days after the first administration of L-NAME), as well as collecting 24-hour urine on day 17-18 of gestation and measuring the level of total protein. Also, we recorded the number of pups produced by each group of pregnant rats.

### Example 4 Experimental results

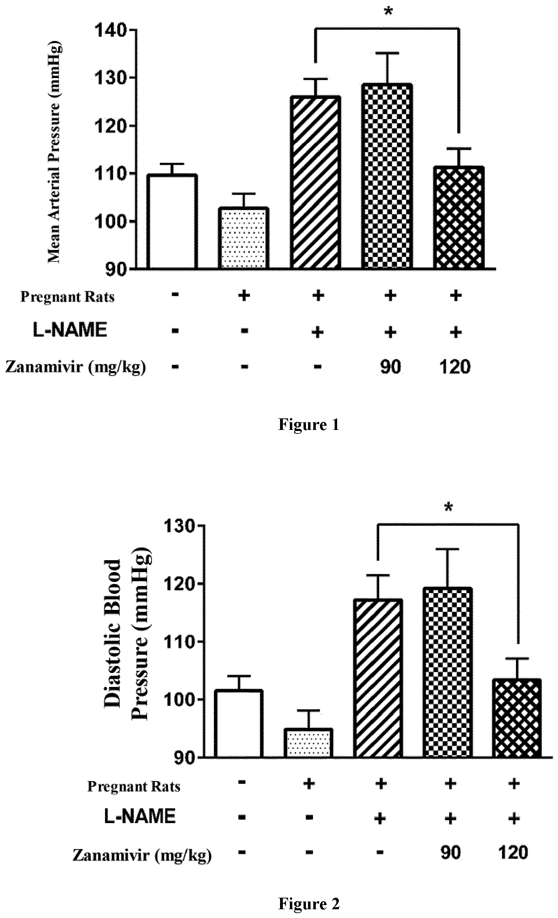

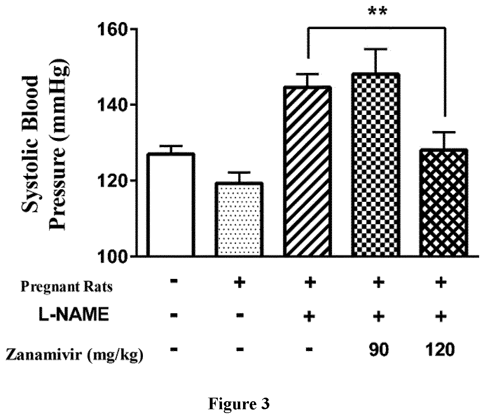

The results of blood pressure assay on day 12 of gestation in pregnant mice (Figures 1-3) showed that the mean arterial, systolic, and diastolic blood pressures were significantly elevated in the pregnant model mice compared with the pregnant control group, predicting that this animal model successfully showed the hypertensive symptoms of pre-eclampsia. Among them, 90 mg/kg zanamivir did not significantly change the blood pressure. However, 120 mg/kg of zanamivir significantly reduced the L-NAME-induced increases in mean arterial, systolic, and diastolic blood pressure in pregnant rats, and the differences were statistically significant.

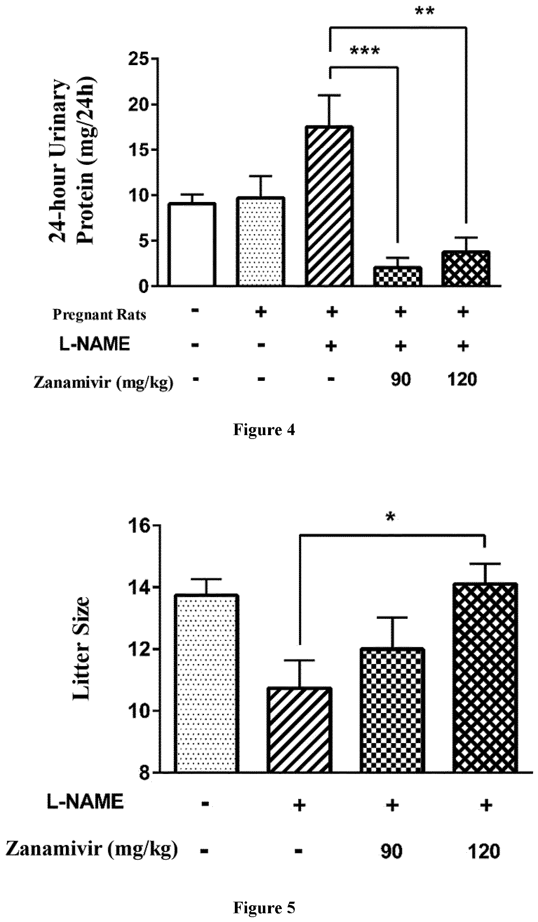

The results of 24-hour total protein measurement in urine on day 17-18 of gestation (Figure 4) showed a significant elevation in 24-hour urine protein in the pregnant model group of mice compared to the pregnant control group, predicting that this animal model successfully showed the proteinuric symptoms of pre-eclampsia. Zanamivir at 90 mg/kg and 120 mg/kg administration significantly reduced the L-NAME-induced urinary protein elevation in pregnant mice, and both were statistically significantly different.

In this experiment, we also recorded the number of pups produced by each group of pregnant rats. As shown in Figure 5, the number of pups produced in the pregnant model group was significantly reduced compared to the pregnant control group. Zanamivir at 90 mg/kg and 120 mg/kg significantly improved the reduction of litter size in pregnant rats caused by L-NAME, with the number of pups in the 120 mg/kg group returning to a level comparable to that of the pregnant control group, with statistically significant differences.

In conclusion, zanamivir significantly improved the symptoms of hypertension and proteinuria in L-NAME-induced preeclamptic pregnant rats and increased the number of litters born. The results suggest that zanamivir has preventive and therapeutic effects on pre-eclampsia, improves maternal hypertension and proteinuria symptoms, and also has the potential to protect fetal development.

## Claims

1. Use of zanamivir in the preparation of drugs for the treatment or prevention of pre-eclampsia, **characterized in that** said zanamivir comprises the compound zanamivir and medically or pharmaceutically acceptable derivatives or salts thereof.

2. The use according to claim 1, **characterized in that** said pharmaceutical dosage form is an injection, an oral formulation or a topical formulation.

3. The use according to claim 2, **characterized in that** said injectables include injectable solutions, powder injections; oral preparations include tablets, capsules, oral liquids, throat or nasal sprays, and topical preparations include ointments, sprays, patches.

4. The use according to any one of claims 1-3, **characterized in that** said treatment or prevention of pre-eclampsia is to improve headache, ophthalmia, malignancy, vomiting, abdominal discomfort, hypertension, proteinuria, edema, liver and kidney function, thrombocytopenia and/or coagulation system abnormalities; elevated creatinine, neuraminidase and sialic acid, and reduced blood NO levels; and improved pregnancy outcomes .

5. A method for treating or preventing pre-eclampsia, comprising administering zanamivir to a patient at risk of or suffering from pre-eclampsia.

6. The method according to claim 5, **characterized in that** said zanamivir dosage form is an injection, an oral formulation or a topical formulation.

7. The method according to claim 6, **characterized in that** said injectables include injectable solutions, powder injections; oral preparations include tablets, capsules, oral liquids, throat or nasal sprays, and topical preparations include ointments, sprays, patches.

8. The method according to any one of claims 5-7, **characterized in that** the method of administration of zanamivir as described in is optionally intramuscular, intravenous, intravenous infusion, preferably intramuscular; administered 1-7 times per week, preferably 1-4 times; more preferably 1-2 times; duration of administration 1-30 days, preferably 1-7 days, more preferably 1-7 days.

9. The method according to any one of claims 5-8, **characterized in that** said treating or preventing pre-eclampsia is to improve headache, ophthalmia, malignancy, vomiting, abdominal discomfort, hypertension, proteinuria, edema, liver and kidney function, thrombocytopenia and/or coagulation system abnormalities ; elevated creatinine, neuraminidase and sialic acid, and reduced blood NO levels; and improving pregnancy outcomes.

10. A drug for the treatment or prevention of pre-eclampsia, **characterized in that** it contains zanamivir.

11. The drug according to claim 10, **characterized in that** said drug dosage form is an injectable, oral formulation.

12. The drug according to claim 11, **characterized in that** said drug dosage form is an injection, an oral formulation or a topical formulation; preferably an injection, an oral formulation; particularly preferably an injection; further preferably an intramuscular injection.

13. The drug according to any one of claims 10-12, **characterized in that** said drug comprises an instruction sheet which states that zanamivir is to be administered 1-7 times per week, preferably 1-4 times; more preferably 1-2 times; for a duration of administration of 1-30 days, preferably 1-7 days, more preferably 1-7 days.

14. The drug according to any one of claims 10-13, **characterized in that** said treating or preventing pre-eclampsia is improving headache, ophthalmia, malignancy, vomiting, abdominal discomfort, hypertension, proteinuria, edema, liver and kidney function, thrombocytopenia and/or coagulation system abnormalities ; elevated creatinine, neuraminidase and sialic acid, reduced blood NO levels; and improving pregnancy outcomes .

15. The method according to any one of claims 5-9 or the drug according to any one of claims 10-14 , **characterized in that** the amount of zanamivir administered or the instructions labeled zanamivir is administered at an amount of 20-40 mg/day.

16. The method or drug according to claim 15, **characterized in that** the amount of zanamivir administered or the instructions label the amount of zanamivir administered as 20 mg/day.

17. The drug according to any one of claims 10-16, further comprising a pharmaceutically acceptable excipient, said pharmaceutically acceptable excipient comprising, but not limited to, a solvent, a co-solvent, a stabilizer, a dispersant, a viscosity modifier, an antioxidant, a sweetener, a binder, a gas generating agent.

18. The method according to any one of claims 5-9 or the drug according to any one of claims 10-17, **characterized in that** zanamivir is the sole active ingredient in the method or drug; or **characterized in that** the method further comprises administering other active ingredients or the drug further comprises other active ingredients; said other active ingredients including, but not limited to, hypotensive agents, protein supplements, anti-vertigo agents, cardiovascular drugs.

19. The use of neuraminidase inhibitors in the preparation of drugs for the treatment or prevention of pre-eclampsia, **characterized in that** said neuraminidase inhibitors comprise neuraminidase inhibitors and medically or pharmaceutically acceptable derivatives or salts thereof.

20. The use according to claim 19, **characterized in that** said pharmaceutical dosage form is an injectable, an oral formulation or a topical formulation; preferably an injectable, an oral formulation; particularly preferably an injectable.

21. The use according to claim 20, **characterized in that** said injectables include injectable solutions, powder injections; oral formulations include tablets, capsules, oral liquids, throat or nasal sprays, and topical formulations include ointments, sprays, patches.

22. The use according to any one of claims 19-21, **characterized in that** said treating or preventing pre-eclampsia is improving headache, ophthalmia, malignancy, vomiting, abdominal discomfort, hypertension, proteinuria, edema, liver and kidney function, thrombocytopenia and/or coagulation system abnormalities; elevated creatinine, neuraminidase and sialic acid, and reduced blood NO levels; and improving pregnancy outcomes.

23. A method of treating or preventing pre-eclampsia, comprising administering a neuraminidase inhibitor to a patient at risk of or suffering from pre-eclampsia.

24. The method according to claim 23, **characterized in that** said neuraminidase inhibitor dosage form is an injectable, an oral formulation or a topical formulation; preferably an injectable, an oral formulation; particularly preferably an injectable.

25. The method according to claim 24, **characterized in that** said injectable agent comprises an injection, a powder injection; an oral preparation comprises a tablet, a capsule, an oral solution, a throat or nasal spray, and a topical preparation comprises an ointment, a spray, a patch.

26. The method according to any one of claims 23-25, **characterized in that** said neuraminidase inhibitor is administered optionally by intramuscular injection, intravenous injection, intravenous infusion, preferably intramuscular; administered 1-7 times per week, preferably 1-4 times; more preferably 1-2 times; and administered for a duration of 1-30 days, preferably 1-7 days, more preferably 1-7 days.

27. The method according to any one of claims 25-26, **characterized in that** said treating or preventing pre-eclampsia is improving headache, ophthalmia, malignancy, vomiting, abdominal discomfort, hypertension, proteinuria, edema, liver and kidney function, thrombocytopenia and/or coagulation system abnormalities; elevated creatinine, neuraminidase and sialic acid, and reduced blood NO levels; and improving pregnancy outcomes.

28. A drug for the treatment or prevention of pre-eclampsia, **characterized in that** it comprises a neuraminidase inhibitor.

29. The drug according to claim 28, **characterized in that** said drug dosage form is an injectable, oral formulation.

30. The drug according to claim 29, **characterized in that** said drug dosage form is an injection, an oral formulation or a topical formulation; preferably an injection, an oral formulation; particularly preferably an injection; further preferably an intramuscular injection.

31. The drug according to any one of claims 28-30, **characterized in that** said drug comprises an instruction sheet which states that the neuraminidase inhibitor is to be administered 1-7 times per week, preferably 1-4 times; more preferably 1-2 times; and the duration of administration is 1-30 days, preferably 1-7 days, more preferably 1-7 days.

32. The drug according to any one of claims 28-31, **characterized in that** said treating or preventing pre-eclampsia is improving headache, ophthalmia, malignancy, vomiting, abdominal discomfort, hypertension, proteinuria, edema, liver and kidney function, thrombocytopenia and/or coagulation system abnormalities; elevated creatinine, neuraminidase and sialic acid, and reduced blood NO levels; and improving pregnancy outcomes.

33. The method according to claims 23-27 or the drug according to any one of claims 28-32, **characterized in that** the neuraminidase inhibitor is administered at a dosage or the specification neuraminidase inhibitor is administered at a dosage of 20-40 mg/day.

34. The method or drug according to claim 15, **characterized in that** the neuraminidase inhibitor dosage or the instructions are labeled with a neuraminidase inhibitor dosage of 20 mg/day.

35. The drug according to any one of claims 28-34, further comprising a pharmaceutically acceptable excipient, said pharmaceutically acceptable excipient comprising, but not limited to, a solvent, a co-solvent, a stabilizer, a dispersant, a viscosity modifier, an antioxidant, a sweetener, a binder, a gas generating agent.

36. The method according to any one of claims 23-27 or the drug according to any one of claims 28-35, **characterized in that** the neuraminidase inhibitor is the sole active ingredient in the method or drug; or wherein the method further comprises administering other active ingredients or the drug further comprises other active ingredients; said other active ingredients including, but not limited to, hypotensive agents, protein supplements, anti-vertigo cardiovascular disease drugs.

37. The use, method or drug according to any one of the foregoing claims, **characterized in that** the neuraminidase inhibitor such as zanamivir is administered at a dosage of 90 mg/kg-120 mg/kg; preferably 120 mg/kg.
